Europäisches Patentamt

European Patent Office

Office européen des brevets

(11) Veröffentlichungsnummer: **0 313 909**
**A2**

## (12) EUROPÄISCHE PATENTANMELDUNG

(21) Anmeldenummer: 88116821.5

(22) Anmeldetag: 11.10.88

(51) Int. Cl.⁴: **C07C 101/28**

(30) Priorität: 24.10.87 DE 3736078

(43) Veröffentlichungstag der Anmeldung:
03.05.89 Patentblatt 89/18

(84) Benannte Vertragsstaaten:
BE CH DE FR GB IT LI NL

(71) Anmelder: HOECHST AKTIENGESELLSCHAFT
Postfach 80 03 20
D-6230 Frankfurt am Main 80(DE)

(72) Erfinder: Erpenbach, Heinz, Dr.
Oberbuschweg 22
D-5000 Köln(DE)
Erfinder: Gehrmann, Klaus, Dr.
Geschwister-Scholl-Strasse 32
D-5042 Erftstadt(DE)
Erfinder: Hörstermann, Peter, Dr.
Theodor-Heuss-Strasse 2
D-5042 Erftstadt(DE)
Erfinder: Jägers, Erhard, Dr.
Hemberger Strasse 75
D-5303 Bornheim(DE)
Erfinder: Kohl, Georg
Rosellstrasse 11
D-5030 Hürth(DE)

(54) Verfahren zur Herstellung von Phenylalanin-n-propylester-hydrochlorid.

(57) Zur Herstellung von Phenylalanin-n-propylester-hydrochlorid setzt man Phenylalanin-hydrochlorid mit n-Propanol im Molverhältnis 1 : (5 bis 20) bei Temperaturen von 80 bis 110°C, vorzugsweise von 85 bis 105°C, und Drucken von 0,1 bis 1,5 bar in Gegenwart von 0,1 bis 0,75 mol HCl/mol Phenylalanin-hydrochlorid als Katalysator und eines Wasserschleppers um, wobei man im Reaktionsgemisch eine Konzentration an Wasserschlepper von 20 bis 50 Gewichts% aufrechterhält.

EP 0 313 909 A2

## Verfahren zur Herstellung von Phenylalanin-n-propylester-hydrochlorid

Die vorliegende Erfindung betrifft ein Verfahren zur Herstellung von Phenylalanin-n-propylester-hydrochlorid durch Umsetzung von Phenylalanin-hydrochlorid mit n-Propanol in flüssiger Phase in Gegenwart von HCl als Katalysator.

Es ist bekannt, D,L-Phenylalanin-propylester durch Umsetzung von D,L-Phenylalanin mit HCl enthaltendem n-Propanol in flüssiger Phase zu erhalten (vergl. "Beilsteins Handbuch der Organischen Chemie", 4. Auflage, 1985, 4. Ergänzungswerk, 14. Band, 3. Teil, Seite 1556).

Bei der bekannten Umsetzung handelt es sich um eine Gleichgewichtsreaktion, so daß der Umsetzungsgrad zum Phenylalanin-n-propylester durch die Gleichgewichtskonstante begrenzt ist. Daher müssen nichtumgesetzte Ausgangsstoffe vom gebildeten Phenylalanin-n-propylester abgetrennt und erneut zur Umsetzung gebracht werden; ein Umstand, welcher bei der Übertragung des Verfahrens in den technischen Maßstab Schwierigkeiten bereitet und daher nachteilig ist.

Um den Umsatz von Phenylalanin zum Phenylalanin-n-propylester zu erhöhen, muß ein bis zu 50 facher molarer Überschuß von n-Propanol, bezogen auf das eingesetzte Phenylalanin, aufgewendet, eine hohe HCl-Konzentration aufrechterhalten und das bei der Umsetzung gebildete Wasser durch azeotrope Destillation gemeinsam mit viel n-Propanol entfernt werden. Von Nachteil ist dabei, daß einerseits das abdestillierte wasserhaltige n-Propanol erst nach aufwendiger Trocknung wieder für die Umsetzung zur Verfügung steht und andererseits unerwünschte Nebenprodukte wie Di-n-propylether bzw. n-Propylchlorid gebildet werden. Bei der Verwendung von Schwefelsäure oder organischen Sulfonsäuren stellen die im Rückstand der Veresterung verbleibenden Katalysatoren ein besonderes Umweltproblem dar, welches nur schwer zu bewältigen ist.

Es ist Aufgabe der vorliegenden Erfindung, ein Verfahren zur Herstellung von Phenylalanin-n-propylester -hydrochlorid aus Phenylalanin-hydrochlorid und n-Propanol anzugeben, welches die genannten Nachteile nicht aufweist, bei welchem vielmehr die Umsetzung bei geringerem n-Propanol-Überschuß und verringerter Menge von HCl als saurem Katalysator in guten Ausbeuten abläuft. Das wird erfindungsgemäß dadurch erreicht, daß man Phenylalanin-hydrochlorid bei Temperaturen von 80 bis 110 °C, vorzugsweise von 85 bis 105 °C, und Drucken von 0,1 bis 1,5 bar mit dem n-Propanol im Molverhältnis 1 : (5 bis 20) in Gegenwart von 0,1 bis 0,75 mol HCl/mol Phenylalanin-hydrochlorid und eines Wasserschleppers umsetzt, wobei man im Reaktionsgemisch eine Konzentration an Wasserschlepper von 20 bis 50 Gewichts% aufrechterhält.

Das Verfahren gemäß der Erfindung kann weiterhin wahlweise auch noch dadurch ausgestaltet sein, daß man

a) als Wasserschlepper Toluol verwendet;

b) als Wasserschlepper 1,2-Dichlorethan verwendet;

c) im Reaktionsgemisch eine HCl-Konzentration von 0,15 bis 1,5 Gewichts% durch Dosierung von 0,1 bis 0,75 mol HCl/mol Phenylalanin-hydrochlorid einstellt und aufrechterhält;

d) das Schleppmittel während der Umsetzung unter einer Rückflußrate von 3 bis 10 als Azeotrop mit Wasser abdestilliert und nach Trennung der Phasen die organische Phase mindestens teilweise ins Reaktionsgemisch zurückführt;

e) nach vollständiger Veresterung das Schleppmittel und den überschüssigen n-Propanol weitgehend abdestilliert, das verbleibende Sumpfprodukt in zusätzlichem Toluol oder Xylol aufnimmt, restliches Propanol durch Destillation entfernt und nach Abkühlen der heißen Lösung daraus das reine, kristalline Phenylalanin-n-propylester-hydrochlorid durch Filtration und anschließende Trocknung gewinnt.

Beim erfindungsgemäßen Verfahren wird durch die Durchführung der Veresterungsreaktion bei Temperaturen von 80 bis 110 °C eine befriedigende Reaktionsgeschwindigkeit bei nur geringfügiger Bildung von n-Propylchlorid erzielt; die n-Propylchlorid-Bildung wird nämlich durch hohe Temperaturen begünstigt.

Beim Verfahren gemäß der Erfindung kann als Ausgangsstoff sowohl Phenylalanin-hydrochlorid direkt als auch Phenylalanin selbst eingesetzt werden, wobei das Phenylalanin im Reaktionsgemisch durch zusätzlich einzubringenden Chlorwasserstoff in das Hydrochlorid überführt wird.

Beispiel 1 (Vergleichsbeispiel)

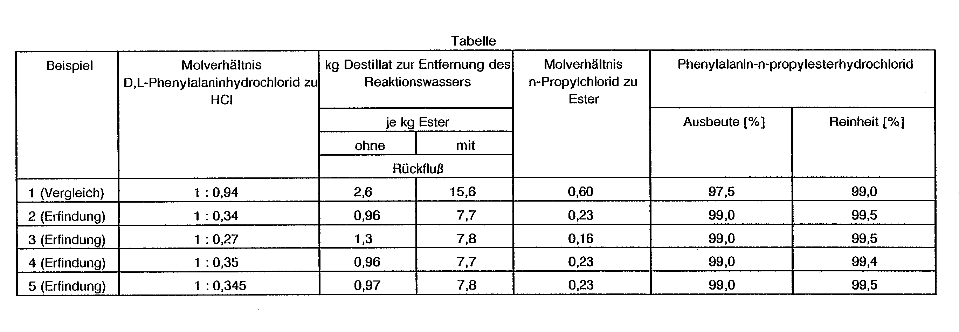

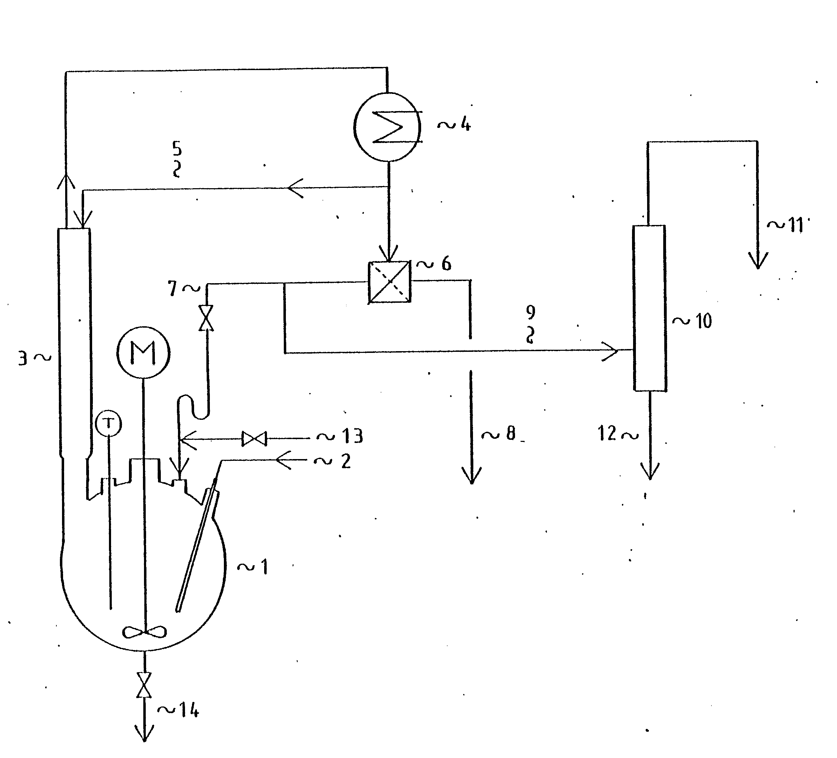

In einen aus Glas bestehenden Rührreaktor 1 (vergl. die Figur) mit innenliegender Heizschlange, welche durch einen über einen Durchlauferhitzer gesteuerten Ölumlauf beheizbar war, und mit einem Füllvolumen von 100 1 wurden 5 Kg D,L-Phenylalanin-hydrochlorid (25 mol) und 40 kg n-Propanol (670 mol) vorgelegt, was einem Molverhältnis von etwa 1 : 27 entspricht. Durch Einleiten von 850 g gasförmiger HCl (23,4 mol) über die Leitung 2 wurde eine HCl-Konzentration von 0,7 Gewichts% in der Reaktionsmischung eingestellt und aufrechterhalten. Dies entspricht einem Molverhältnis D,L-Phenylalanin-hydrochlorid: HCl = 1 : 0,94. Das bei der Umsetzung freiwer dende Wasser wurde gemeinsam mit n-Propanol, welches gleichzeitig als Schleppmittel diente, als azeotropes Gemisch über Kopf einer auf den Rührreaktor 1 aufgesetzten Kolonne 3 bei einer Reaktortemperatur von 98°C und Normaldruck abdestilliert. Durch Kondensation im Kühler 4 wurde unter Einstellung eines über eine Leitung 5 geführten konstanten Rückflusses das Azeotrop entnommen. Die Reaktionszeit bis zur vollständigen Veresterung des D,L-Phenylalanin-hydrochlorids betrug 6 Stunden, wobei dies nur erreichbar war, wenn pro kg erzeugten Esters unter einer Rückflußrate von 5 eine Destillatmenge von 2,6 kg (mit 4,7 Gewichts% Wasser, 1 Gewichts% HCl, 7,2 Gewichts% n-Propylchlorid, Rest n-Propanol) abgezogen wurde. Die Abtrennung von n-Propylchlorid und Wasser aus diesem Destillat ist nur schwer durchführbar und äußerst kostenaufwendig.

Nach vollständiger Veresterung des D,L-Phenylalanin-hydrochlorids wurde das überschüssige n-Propanol bei Normaldruck abdestilliert. Nach Erreichen einer Sumpftemperatur von 102°C wurde die Beheizung kurzzeitig abgestellt und es wurden 25 kg Xylol über die Leitung 13 zugesetzt. Nun wurde das restlich n-Propanol bei 280 mbar abdestilliert, bevor die heiße Xylollösung über die Leitung 14 in Kristallisationsgefäße abgelassen wurde. Beim Erkalten unter Rühren fiel reines D,L-Phenylalanin-n-propylester-hydrochlorid aus, von dem abfiltriert wurde. Nach dem Trocknen lagen 5,9 kg D,L-Phenylalanin-n-propylester-hydrochlorid (24,2 mol) in 99 %iger Reinheit vor, was einer Esterausbeute von 97,5 % entspricht. Das Molverhältnis von gebildetem n-Propylchlorid zu D,L-Phenylalanin-n-propyl-ester-hydrochlorid betrug 0,6.

Beispiel 2 (gemäß der Erfindung)

In den im Beispiel 1 verwendeten Rührreaktor 1 (vergl. die Figur) wurden 10 kg D, L-Phenylalanin-hydrochlorid (50 mol), 26,4 kg n-Propanol (440 mol), 19,4 kg Toluol und 0,5 kg Salzsäure (31 %ig) vorgelegt, was einem Molverhältnis D,L-Phenylalanin-hydrochlorid : n-Propanol von 1 : 8,8 entspricht. Während der Veresterung wurden 465 g gasförmiger HCl (12,75 mol) über die Leitung 2 zugeführt und so eine HCl-Konzentration von 0,7 Gewichts% in der Reaktionsmischung eingestellt und aufrechterhalten. Die insgesamt zugesetzte HCl-Menge entspricht einem Molverhältnis D,L-Phenylalanin-hydrochlorid : HCl von 1 : 0,34. Die Reaktionstemperatur betrug 96°C, die Zeit bis zur vollständigen Veresterung 8 Stunden. Innerhalb dieses Zeitraumes wurden über die dem Reaktor 1 aufgesetzte Kolonne 3 unter einer Rückflußrate von 7 0,96 kg Destillat je kg produzierten Esters abgezogen. Das im Kühler 4 anfallende Destillat wurde im Abscheider 6 in zwei Phasen getrennt; und zwar in 18 % wäßrige und 82 % organische Phase. Während die wäßrige Phase, welche aus ca. 23 Gewichts% n-Propanol, 1 Gewichts% Toluol, 4,5 Gewichts% HCl, Rest Wasser bestand, über die Leitung 8 abgezogen wurde, wurde die organische Phase bis zum Erreichen eines Gehaltes von ca. 14 Gewichts% n-Propylchlorid über die Leitung 7 in den Reaktor 1 zurückgeführt. Anschließend wurden etwa 70 % der insgesamt anfallenden organischen Phase, welche neben der genannten Menge n-Propylchlorid noch 2,2 Gewichts% Wasser, 21 Gewichts% Propanol, Rest Toluol enthielt, über die Leitung 9 einer Trennstufe 10 zugeführt und bei Normaldruck unter einer Rückflußrate von 6 bis zu einer Sumpftemperatur von 74°C und einer Kopftemperatur von 50°C destillativ zerlegt. Dabei fielen via Leitung 11 0,9 kg Destillat mit 97,5 Gewichts% n-Propylchlorid (11,2 mol) an, während das über die Leitung 12 abgezogene, aus Propanol und Toluol bestehende Sumpfprodukt bei folgenden Ansatz wiederverwendet wurde. Nach vollständiger Veresterung des eingesetzten D,L-Phenylalanin-hydrochlorids wurden das restliche Toluol und das überschüssige n-Propanol über Kopf der Kolonne 3 abdestilliert und nach Kondensation im Kühler 4 über die Leitung 8 abgezogen. Nach Erreichen einer Sumpftemperatur von ca. 102°C wurde die Beheizung unterbrochen und über die Leitung 13 Xylol (39 kg) in den Reaktor 1 eingefüllt. Anschließend wurden bei einem Druck von 280 mbar und einer Rückflußrate von 1 das Toluol und das restliche n-Propanol abdestilliert. Das erhaltene Destillat wurde ohne weitere Behandlung beim folgenden Ansatz verwendet.

Die heiße Xylollösung wurde in Kristallisationsgefäße abgelassen. Beim Erkalten unter Rühren kristallisierte reines D,L-Phenylalanin-n-propylester-hydrochlorid aus, von dem abfiltriert wurde. Nach Trocknung wurden 12 kg D,L-Phenylalanin-n-propylester-hydrochlorid (49,5 mol) in 99,5 %iger Reinheit erhalten, was einer Esterausbeute von 99 % entspricht. Das Molverhältnis von gebildetem n-Propylchlorid zu D,L-Phenylalanin-n-propylester-hydrochlorid betrug 0,23.

3

Beispiel 3 (gemäß der Erfindung)

In den im Beispiel 1 verwendeten Rührreaktor 1 (vergl. die Figur) wurden 10 kg D,L-Phenylalanin-hydrochlorid (50 mol), 32 kg n-Propanol (530 mol), 20 kg 1,2-Dichlorethan und 0,5 kg Salzsäure (31 %ig) vorgelegt, was einem Molverhältnis D,L-Phenylalanin-hydrochlorid : n-Propanol von 1 : 10,6 entspricht. Während der Veresterung wurden 325 g gasförmiger HCl (9 mol) über die Leitung 2 zugeführt und so eine HCl-Konzentration von 0,7 Gewichts% in der Reaktionsmischung eingestellt und aufrechterhalten. Die insgesamt zugesetzte HCl-Menge entspricht einem Molverhältnis D,L-Phenylalanin-hydrochlorid : HCl von 1 : 0,27. Die Reaktionstemperatur betrug 94° C, die Zeit bis zur vollständigen Veresterung 6,5 Stunden. Innerhalb dieses Zeitraumes wurden über die dem Reaktor 1 aufgesetzte Kolonne 3 unter einer Rückflußrate von 5 1,3 kg Destillat je kg produziertem Esters abgezogen. Das im Kühler 4 anfallende Destillat wurde im Abscheider 6 in zwei Phasen getrennt; und zwar in 12 % wäßrige und 88 % organische Phase. Während die wäßrige Phase, welche aus ca. 18 Gewichts% n-Propanol, 7 Gewichts% HCl, Rest Wasser bestand, über die Leitung 8 abgezogen wurde, wurde die organische Phase bis zum Erreichen eines Gehaltes von ca. 9 Gewichts% n-Propylchlorid über die Leitung 7 in den Reaktor 1 zurückgeführt. Anschließend wurden etwa 50 % der insgesamt anfallenden organischen Phase, welche neben der genannten Menge N-Propylchlorid noch 7 Gewichts% Propanol, Rest 1,2-Dichlorethan enthielt, über die Leitung 9 einer Trennstufe 10 zugeführt, bei Normaldruck unter einer Rückflußrate von 6 bis zu einer Sumpftemperatur von 77° C und einer Kopftemperatur von 50° C destillativ zerlegt. Dabei fielen via Leitung 11 0,65 kg Destillat mit 96 Gewichts% n-Propylchlorid (7,9 mol) an, während das über die Leitung 12 abgezogene, aus Propanol und 1,2-Dichlorethan bestehende Sumpfprodukt beim folgenden Ansatz wiederverwendet wurde. Nach vollständiger Veresterung des eingesetzten D,L-Phenylalanin-hydrochlorids wurden das restliche 1,2-Dichlorethan und das überschüssige n-Propanol über Kopf der Kolonne 3 abdestilliert und nach Kondensation im Kühler 4 über die Leitung 8 abgezogen.Nach Erreichen einer Sumpftemperatur von ca. 102° C wurde die Beheizung unterbrochen und über die Leitung 13 Xylol (39 kg) in den Reaktor 1 eingefüllt. Anschließend wurden bei einem Druck von 280 mbar und einer Rückflußrate von 1 das 1,2-Dichlorethan und das restliche n-Propanol abdestilliert. Das erhaltene Destillat wurde ohne weitere Behandlung beim folgenden Ansatz verwendet.

Die heiße Xylollösung wurde in Kristallisationsgefäße abgelassen. Beim Erkalten unter Rühren kristallisierte reines D,L-Phenylalanin-n-propylester-hydrochlorid aus, von dem abfiltriert wurde. Nach Trocknung wurden 12 kg D,L-Phenylalanin-n-propylester-hydrochlorid (49,5 mol) in 99,5 %iger Reinheit erhalten, was einer Esterausbeute von 99 % entspricht. Das Molverhältnis von gebildetem n-Propylchlorid zu D,L-Phenylalanin-n-propylester-hydrochlorid betrug 0,16.

Beispiel 4 (gemäß der Erfindung)

In dem im Beispiel 1 verwendeten Rührreaktor 1 (vergl. die Figur) wurden 10 kg D,L-Phenylalanin-hydrochlorid (50 mol), 28,2 kg n-Propanol (470 mol), 27,8 kg Toluol und 0,5 kg Salzsäure (31%ig) vorgelegt, was einem Molverhältnis D,L-Phenylalanin-hydrochlorid zu n-Propanol von 1 : 9,4 entspricht. Während der Veresterung wurden 480 g gasförmiger HCl (13,15 mol) über die Leitung 2 zugeführt und so eine HCl-Konzentration in der Reaktionsmischung von 0,7 Gewichts% eingestellt und aufrechterhalten. Die insgesamt zugesetzte HCl-Menge entspricht einem Molverhältnis D,L-Phenylalanin-hydrochlorid zu HCl von 1 : 0,35. Die Reaktionstemperatur betrug 96° C, die Zeit bis zur vollständigen Veresterung 7 Stunden. Innerhalb dieses Zeitraums wurde über die dem Reaktor 1 aufgesetzte Kolonne 3 unter einer Rückflußrate von 7 eine Destillatmenge von 0,96 kg/kg produzierten Esters abgezogen. Das im Kühler 4 anfallende Destillat wurde im Abscheider 6 in zwei Phasen getrennt; und zwar in 18 % wäßrige und 82 % organische Phase. Während die wäßrige Phase, welche aus ca. Gewichts% n-Propanol, 1 Gewichts% Toluol, 4,5 Gewichts% HCl, Rest Wasser bestand, über die Leitung 8 abgezogen wurde, wurde die organische Phase bis zum Erreichen eines Gehaltes von ca. 14 Gewichts% n-Propylchlorid über die Leitung 7 in den Reaktor 1 zurückgeführt. Anschließend wurden etwa 70 % der insgesamt anfallenden organischen Phase, welche neben der genannten Menge n-Propylchlorid noch 2,2 Gewichts% Wasser, 21 Gewichts% n-Propanol, Rest Toluol enthielt, über die Leitung 9 einer Trennstufe 10 zugeführt und bei Normaldruck unter einer Rückflußrate von 6 bis zu einer Sumpftemperatur von 74° C und einer Kopftemperatur von 50° C destillativ zerlegt. Dabei fielen über Leitung 11 0,925 kg Destillat mit 97 Gewichts% n-Propylchlorid (11,6 mol) an, während das über die Leitung 12 abgezogene, aus Propanol und Toluol bestehende Sumpfprodukt beim folgenden Ansatz wieder verwendet wurde. Nach vollständiger Veresterung des eingesetzten D,L-Phenylalanin-hydrochlorids wurden Toluol und überschüssiges n-Propanol über Kopf der Kolonne 3 abde-

4

stilliert und nach Kondensation im Kühler 4 über die Leitung 8 abgezogen. Nach Erreichen einer Sumpftemperatur von ca. 102°C wurde die Beheizung unterbrochen und über die Leitung 13 zusätzliches Toluol (35 kg) in den Reaktor 1 eingefüllt. Anschließend wurde bei einem Druck von 280 mbar und einer Rückflußrate von 1 das restliche n-Propanol azeotrop mit Toluol abdestilliert. Des erhaltene Destillat wurde ohne weitere Behandlung beim folgenden Ansatz verwendet.

Die heiße Toluollösung wurde über Leitung 14 Kristallisationsgefäße abgelassen. Beim Erkalten unter Rühren kristallisierte reines D,L-Phenylalanin-n-propylester-hydrochlorid aus, von dem abfiltriert wurde. Nach Trocknung wurden 12 kg D,L-Phenylalanin-n-propylester-hydrochlorid (49,5 mol) in 99,4 %iger Reinheit erhalten, was einer Esterausbeute von 99 % entspricht. Das Molverhältnis von gebildetem n-Propylchlorid zu D,L-Phenylalanin-n-propylester-hydrochlorid betrug 0,23.


Beispiel 5 (gemäß der Erfindung)

in den im Beispiel 1 verwendeten Rührreaktor 1 (vgl. die Figur) wurden 10 kg L-Phenylalaninhydrochlorid (50 mol), 29,4 kg n-Propanol (490 mol), 20,2 kg Toluol und 0,5 kg Salzsäure (31 %ig) vorgelegt, was einem Molverhältnis L-Phenylalanin-hydrochlorid zu n-Propanol von 1 : 9,8 entspricht. Während der Veresterung wurden 475 g gasförmiger HCl (13 mol) über die Leitung 2 zugeführt und so eine HCl-Konzentration von 0,7 Gewichts% in der Reaktionsmischung eingestellt und aufrechterhalten. Die insgesamt zugesetzte HCl-Menge entspricht einem Molverhältnis L-Phenylalanin-hydrochlorid zu HCl von 1 : 0,345. Die Reaktionstemperatur betrug 96°C, die Zeit bis zur vollständigen Veresterung 7 Stunden. Innerhalb dieses Zeitraumes wurden über die dem Reaktor 1 aufgesetzte Kolonne 3 unter einer Rückflußrate von 7 eine Destillatmenge von 0,97 kg/kg produzierten Esters abgezogen. Das im Kühler 4 anfallende Destillat wurde im Abscheider 6 in zwei Phasen getrennt; und zwar in 18 % wäßrige und 82 % organische Phase. Während die wäßrige Phase, welche aus ca. 23 Gewichts% n-Propanol, 1 Gewichts% Toluol, 4,5 Gewichts% HCl, Rest Wasser bestand, über die Leitung 8 abgezogen wurde, wurde die organische Phase bis zum Erreichen eines Gehaltes von ca. 14 Gewichts% n-Propylchlorid über die Leitung 7 in den Reaktor 1 zurückgeführt. Anschließend wurden etwa 70 % der insgesamt anfallenden organischen Phase, welche neben der genannten Menge n-Propylchlorid noch ca. 2,2 Gewichts% Wasser, 21 Gewichts% Propanol, Rest Toluol enthielt, über die Leitung 9 einer Trennstufe 10 zugeführt und bei Normaldruck unter einer Rückflußrate von 6 bis zu einer Sumpftemperatur von 74°C und einer Kopftemperatur von 50°C destillativ zerlegt. Dabei fielen über Leitung 11  0,92 kg Destillat mit 97,2 Gewichts% n-Propylchlorid (11,4 mol) an, während das über die Leitung 12 abgezogene, aus Propanol und Toluol bestehende Sumpfprodukt beim folgenden Ansatz wieder verwendet wurde.

Nach vollständiger Veresterung des eingesetzten L-Phenylalanin-hydrochlorids wurden Toluol und das überschüssige n-Propanol über Kopf der Kolonne 3 abdestilliert und nach Kondensation im Kühler 4 über die Leitung 8 abgezogen. Nach Erreichen einer Sumpftemperatur von ca. 102°C wurde die Beheizung unterbrochen und über die Leitung 13 Xylol (38 kg) in den Reaktor 1 eingefüllt. Anschließend wurde bei einem Druck von 280 mbar und einer Rückflußrate von 1 das Toluol und das restliche Propanol abdestilliert. Das erhaltene Destillat wurde ohne weitere Behandlung beim folgenden Ansatz verwendet.

Die heiße Xylollösung wurde in Kristallisationsgefäße abgelassen. Beim Erkalten unter Rühren kristallisierte reines L-Phenylalanin-n-propylester-hydrochlorid aus, von dem abfiltriert wurde. Nach Trocknung wurden 12 kg L-Phenylalanin-n-propylester-hydrochlorid (49,5 mol) in 99,5 %iger Reinheit erhalten, was einer Esterausbeute von 99 % entspricht. Das Molverhältnis von gebildetem n-Propylchlorid zu L-Phenylalanin-n-propylester-hydrochlorid betrug 0,23.

Beim erfindungsgemäßen Verfahren ist die Abtrennung des n-Propylchlorids aus der organischen Phase völlig unproblematisch, während die Abtrennung von n-Propylchlorid und Wasser aus dem beim Vergleichsbeispiel anfallenden Destillat nur schwer durchführbar und äußerst kostenaufwendig ist.

Das beim Verfahren gemäß der Erfindung insgesamt anfallende, nichtumgesetzte n-Propanol kann ohne eine Trocknungsstufe gemeinsam mit dem Schleppmittel für einen neuen Veresterungsansatz verwendet werden, während das n-Propanol des Vergleichsbeispiels mit Wasser und n-Propylchlorid verunreinigt ist.

Die mit den erfindungsgemäßen Beispielen gegenüber dem Vergleichsbeispiel erzielten Vorteile sind aus der folgenden Tabelle ersichtlich.

**Ansprüche**

1. Verfahren zur Herstellung von Phenylalanin-n-propylester-hydrochlorid durch Umsetzung von Phenylalanin-hydrochlorid mit n-Propanol in flüssiger Phase in Gegenwart von HCl als Katalysator, dadurch gekennzeichnet, daß man Phenylalanin-hydrochlorid bei Temperaturen von 80 bis 110° C, vorzugsweise von 85 bis 105° C, und Drucken von 0,1 bis 1,5 bar mit dem n-Propanol im Molverhältnis 1 : (5 bis 20) in Gegenwart von 0,1 bis 0,75 mol HCl/mol Phenylalanin-hydrochlorid und eines Wasserschleppers umsetzt, wobei man im Reaktionsgemisch eine Konzentration an Wasserschlepper von 20 bis 50 Gewichts% aufrechterhält.

2. Verfahren nach Anspruch 1, dadurch gekennzeichnet, daß man als Wasserschlepper Toluol verwendet.

3. Verfahren nach Anspruch 1 dadurch gekennzeichnet, daß man als Wasserschlepper 1,2-Dichlorethan verwendet.

4. Verfahren nach mindestens einem der Ansprüche 1 bis 3, dadurch gekennzeichnet, daß man im Reaktionsgemisch eine HCl-Konzentration von 0,15 bis 1,5 Gewichts% durch Dosierung von 0,1 bis 0,75 mol HCl/mol Phenylalanin-hydrochlorid einstellt und aufrechterhält.

5. Verfahren nach mindestens einem der Ansprüche 1 bis 4, dadurch gekennzeichnet, daß man das Schleppmittel während der Umsetzung unter einer Rückflußrate von 3 bis 10 als Azeotrop mit Wasser abdestilliert und nach Trennung der Phasen die organische Phase mindestens teilweise ins Reaktionsgemisch zurückführt.

6. Verfahren nach mindestens einem der Ansprüche 1 bis 5, dadurch gekennzeichnet, daß man nach vollständiger Veresterung das Schleppmittel und das überschüssige n-Propanol weitgehend abdestilliert, das verbleibende Sumpfprodukt in zusätzlichem Toluol oder Xylol aufnimmt, restliches Propanol durch Destillation entfernt und nach Abkühlen der heißen Lösung daraus das reine kristalline Phenylalanin-n-propylester-hydrochlorid durch Filtration und anschließende Trocknung gewinnt.

Tabelle

| Beispiel | Molverhältnis D,L-Phenylalaninhydrochlorid zu HCl | kg Destillat zur Entfernung des Reaktionswassers | | Molverhältnis n-Propylchlorid zu Ester | Phenylalanin-n-propylesterhydrochlorid | |
|---|---|---|---|---|---|---|
| | | je kg Ester | | | Ausbeute [%] | Reinheit [%] |
| | | ohne | mit | | | |
| | | Rückfluß | | | | |
| 1 (Vergleich) | 1 : 0,94 | 2,6 | 15,6 | 0,60 | 97,5 | 99,0 |
| 2 (Erfindung) | 1 : 0,34 | 0,96 | 7,7 | 0,23 | 99,0 | 99,5 |
| 3 (Erfindung) | 1 : 0,27 | 1,3 | 7,8 | 0,16 | 99,0 | 99,5 |
| 4 (Erfindung) | 1 : 0,35 | 0,96 | 7,7 | 0,23 | 99,0 | 99,4 |
| 5 (Erfindung) | 1 : 0,345 | 0,97 | 7,8 | 0,23 | 99,0 | 99,5 |